(19)

Europäisches Patentamt
European Patent Office
Office européen des brevets

(11) **EP 3 127 608 A1**

(12) **EUROPEAN PATENT APPLICATION**
published in accordance with Art. 153(4) EPC

(43) Date of publication:
**08.02.2017 Bulletin 2017/06**

(21) Application number: **15772755.3**

(22) Date of filing: **06.03.2015**

(51) Int Cl.:
***B01J 23/30*** (2006.01)      ***B01J 37/04*** (2006.01)
***C07C 253/26*** (2006.01)     ***C07C 255/08*** (2006.01)
***C07B 61/00*** (2006.01)

(86) International application number:
**PCT/JP2015/056744**

(87) International publication number:
**WO 2015/151726 (08.10.2015 Gazette 2015/40)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA**

(30) Priority: **31.03.2014 JP 2014074011**

(71) Applicant: **ASAHI KASEI KABUSHIKI KAISHA Tokyo 101-8101 (JP)**

(72) Inventors:
• ISHII, Yusuke
  **Tokyo 101-8101 (JP)**
• OKADA, Kazushi
  **Tokyo 101-8101 (JP)**

(74) Representative: **dompatent von Kreisler Selting Werner - Partnerschaft von Patent- und Rechtsanwälten mbB Deichmannhaus am Dom Bahnhofsvorplatz 1 50667 Köln (DE)**

(54) **METHOD FOR PRODUCING OXIDE CATALYST AND METHOD FOR PRODUCING UNSATURATED NITRILE**

(57)     There is provided a method for producing an oxide catalyst to be used for production of an unsaturated nitrile, the method including a step (a) of preparing an aqueous mixed liquid (A) containing Mo, V and Sb, a step (b) of mixing the aqueous mixed liquid (A), a carrier component and a Nb raw material at 20°C or more and 80°C or less to obtain an aqueous mixed liquid (B), a step (c) of drying the aqueous mixed liquid (B) to obtain a dried powder, and a step (d) of calcining the dried powder to obtain an oxide catalyst, wherein the aqueous mixed liquid (B) has a solid metal component concentration of 2.0 to 8.0% by mass.

**EP 3 127 608 A1**

**Description**

Technical Field

The present invention relates to a method for producing an oxide catalyst, and a method for producing an unsaturated nitrile using the silica-supported oxide catalyst.

Background Art

At the present day, an unsaturated nitrile which is generally available commercially is mainly produced industrially by the catalytic ammoxidation reaction of olefin, ammonia, and oxygen. On the other hand, in recent years, a method for subjecting an alkane such as propane or isobutane as a feed stock in place of the olefin to a vapor-phase catalytic ammoxidation reaction to produce a corresponding unsaturated nitrile has attracted much attention. Various catalysts used in the case have also been proposed.

Patent Literature 1 describes a method for producing a catalyst which contains antimony, exhibiting little escaping, gives a high yield of an unsaturated nitrile, and moreover gives a high space time yield, as a catalyst for the vapor-phase catalytic oxidation or vapor-phase catalytic ammoxidation of propane or isobutane.

Patent Literature 2 describes a method for producing a silica-supported catalyst used when propane or isobutane is subjected to a vapor-phase catalytic ammoxidation reaction to produce an unsaturated nitrile, or the propane or isobutane is subjected to a vapor-phase catalytic oxidation reaction to produce an unsaturated carboxylic acid, wherein the catalyst is supported on 20 to 60% by mass of a silica, and satisfies a pore volume of 0.15 $cm^3$/g or larger; and a powder silica having an average primary particle diameter of 50 nm or smaller is used as at least a part of a silica raw material.

Patent Literature 3 describes a method for producing an oxide catalyst improved in the catalyst performance in a vapor-phase catalytic oxidation reaction or vapor-phase catalytic ammoxidation reaction of propane or isobutane by optimizing the charging composition besides providing a removal step of protrusions present on the particle surface.

Citation List

Patent Literature

Patent Literature 1: Japanese Patent Laid-Open No. 2000-70714
Patent Literature 2: Japanese Patent Laid-Open No. 2002-219362
Patent Literature 3: International Publication No. WO2012/090979

Summary of Invention

Technical Problem

In conventional methods for preparing the catalyst, however, catalyst production steps are complicated and the industrial catalyst production is not easy.

The present invention has been accomplished in view of the above-mentioned problems, and provides a method for producing an oxide catalyst exhibiting a higher performance than conventional ones, without needing the introduction of complicated steps and the variations of facilities, and a method for producing an unsaturated nitrile.

Solution to Problem

As a result of studies by the present inventors, it has become clear that even if the compositions of oxide catalysts are identical, if the solid component concentrations of aqueous mixed liquids when the oxide catalysts are adjusted are different, a difference in the performance of the catalysts is caused. As a result of further exhaustive studies on a method for producing an oxide catalyst, it has been found that by adjusting concentrations (solid metal component concentrations) of metal components excluding a carrier to an aqueous mixed liquid in predetermined ranges, a silica-supported catalyst in which the metal components are uniformly dispersed can be produced, and this finding has led to the present invention.

That is, the present invention is as follows.

[1] A method for producing an oxide catalyst to be used for production of an unsaturated nitrile, the method comprising:

a step (a) of preparing an aqueous mixed liquid (A) comprising Mo, V and Sb;
a step (b) of mixing the aqueous mixed liquid (A), a carrier component and a Nb raw material at 20°C or more and 80°C or less to obtain an aqueous mixed liquid (B);
a step (c) of drying the aqueous mixed liquid (B) to obtain a dried powder; and
a step (d) of calcining the dried powder to obtain an oxide catalyst,

wherein the aqueous mixed liquid (B) has a solid metal component concentration of 2.0 to 8.0% by mass.

[2] The method for producing the oxide catalyst according to [1], wherein the aqueous mixed liquid (B) has a solid component concentration of 6.0 to 20% by mass.

[3] The method for producing the oxide catalyst according to [1] or [2], wherein the carrier component comprises a silica; and the amount of the silica mixed is 20 to 70% by mass in terms of $SiO_2$ based on the total amount of the oxide catalyst.

[4] The method for producing the oxide catalyst according to any one of [1] to [3], wherein the oxide catalyst comprises a metal component represented by the following composition formula (1):

$$Mo_1V_aSb_bNb_cW_dZ_eO_n \text{ ...} \qquad (1),$$

wherein the component Z is at least one element selected from the group consisting of La, Ce, Pr, Yb, Y, Sc, Sr, and Ba; a, b, c, d, e, and n represent atomic ratios of each element and satisfy $0.10 \leq a < 0.20$, $0.15 \leq b \leq 0.30$, $0.010 \leq c \leq 0.50$, $0 \leq d \leq 0.40$, and $0 \leq e \leq 0.20$.

[5] The method for producing the oxide catalyst according to [4], wherein a and b in the composition formula (1) satisfy $0.5 \leq a/b \leq 0.98$.

[6] A method for producing an unsaturated nitrile, comprising a step of subjecting propane or isobutane to a vapor-phase catalytic oxidation reaction or a vapor-phase catalytic ammoxidation reaction with an oxide catalyst produced by the method for producing the oxide catalyst according to any one of [1] to [5] to produce a corresponding unsaturated nitrile.

Advantageous Effects of Invention

The present invention can provide a method for producing an oxide catalyst giving a higher yield of an unsaturated nitrile than conventional ones, without needing the introduction of complicated steps and the variations of facilities, and a method for producing the unsaturated nitrile.

Description of Embodiments

Hereinafter, a mode for carrying out the present invention (hereinafter, merely referred to as "present embodiment") will be described in detail. The following present embodiment is given in order to illustrate the present invention. The present invention should not be construed to be limited to the following contents. The present invention may be carried out while making appropriate modification within the scope of the invention.

[Oxide Catalyst]

A method for producing an oxide catalyst to be used for production of an unsaturated nitrile, according to the present embodiment, the method comprising:

a step (a) of preparing an aqueous mixed liquid (A) comprising Mo, V and Sb;
a step (b) of mixing the aqueous mixed liquid (A), a carrier component and a Nb raw material, and stirring the mixture at 20°C or more and 80°C or less to obtain an aqueous mixed liquid (B);
a step (c) of drying the aqueous mixed liquid (B) to obtain a dried powder; and
a step (d) of calcining the dried powder to obtain an oxide catalyst,

wherein the aqueous mixed liquid (B) has a solid metal component concentration of 2.0 to 8.0% by mass.

[Step (a)]

In the step (a), an aqueous mixed liquid (A) comprising Mo, V and Sb is prepared. Specifically, a raw material comprising Mo (also referred to as a Mo raw material), a raw material comprising V (also referred to as a V raw material), and a raw material comprising Sb (also referred to as an Sb raw material) are mixed to prepare the aqueous mixed liquid (A). Here, the mixing method is not particularly limited, and known mixing means can be used.

The raw material comprising Mo (hereinafter, also referred to as "Mo raw material") is not particularly limited, but specifically, ammonium heptamolybdate $[(NH_4)_6Mo_7O_{24} \cdot 4H_2O]$, molybdenum trioxide $[MoO_3]$, phosphomolybdic acid $[H_3PMo_{12}O_{40}]$, silicomolybdic acid $[H_4SiMo_{12}O_{40}]$, and molybdenum pentachloride $[MoC_{15}]$ or the like can be used. Among them, ammonium heptamolybdate $[(NH_4)_6Mo_7O_{24} \cdot 4H_2O]$ is particularly preferable.

The raw material comprising V (hereinafter, also referred to as "V raw material") is not particularly limited. Specifically, ammonium metavanadate $[NH_4VO_3]$, vanadium pentoxide $[V_2O_5]$, and vanadium chloride $[VCl_4, VCl_3]$ or the like can be used. Among them, ammonium metavanadate $[NH_4VO_3]$ is particularly preferable.

The raw material comprising Sb (hereinafter, also referred to as "Sb raw material") is not particularly limited. Specifically, antimony oxide $[Sb_2O_3, Sb_2O_5]$, antimonious acid $[HSbO_2]$, antimonic acid $[HSbO_3]$, ammonium antimonate $[(NH_4)SbO_3]$, antimony chloride $[Sb_2Cl_3]$, and an organic acid salt such as a tartrate of antimony, and metal antimony or the like can be used. Among them, diantimony trioxide $[Sb_2O_3]$ is particularly preferable.

[Step (b)]

In the step (b), the aqueous mixed liquid (A), a carrier component and a Nb raw material are mixed at 20°C or more and 80°C or less to obtain an aqueous mixed liquid (B). Here, the mixing method is not particularly limited, and known mixing means can be used.

The carrier component is not particularly limited, but examples thereof include silica, alumina, zirconia and titania. The carrier component may be used singly or concurrently in two or more. As the carrier component, especially preferable is silica. By using a silica, a catalyst comprising the silica, preferably a silica-supported catalyst can be obtained. The raw material of the silica is not particularly limited, but specifically, a silica sol can be used, and a powder silica can also be used for a part or the whole amount of the silica raw material.

In the step (b), in the case of using a silica as the carrier component, the amount mixed is preferably 20 to 70% by mass in terms of $SiO_2$ based on the total amount of the oxide catalyst, more preferably 40 to 65% by mass, and still more preferably 40 to 60% by mass. The content of the silica contained in the oxide catalyst is 20% by mass or more, and thereby the strength of the catalyst tends to be further improved; and when 70% by mass or less, and thereby the catalyst tends to have higher activity.

The raw material comprising Nb (hereinafter, also referred to as "Nb raw material") is not particularly limited. Specifically, niobic acid, an inorganic niobate and an organic niobate can be used. Particularly, niobic acid is preferable. Niobic acid is represented by $Nb_2O_5 \cdot nH_2O$, and is denoted also as niobium hydroxide or a niobium oxide compound.

Further, water is preferably added to the Nb raw material. At this time, from the viewpoint of stabilizing the Nb raw material, and the like, the ratio (mol/kg) of the water added to Nb is preferably 0.1 to 10, and more preferably 0.3 to 5.

The Nb raw material may further comprise an organic acid salt or a free organic acid. The organic acid is not particularly limited, but is preferably oxalic acid. The molar ratio of organic acid/niobium is preferably 1 to 4.

A method for mixing a Nb raw material, water and an organic acid is not particularly limited, and these may be mixed in any order. The mixing may be performed at any temperature as long as the temperature is a temperature at which a Nb aqueous solution does not freeze or more, and is a temperature at which the Nb aqueous solution does not boil or less. From the viewpoint of the operability and the like, the mixing is preferably performed at room temperature.

A hydrogen peroxide water is preferably added to the Nb aqueous solution. At this time, from the viewpoints of forming a complex with a Nb raw material to stabilize the Nb raw material in a dissolved state, of properly adjusting the oxidation/reduction state of the catalyst-constituting element, and of making the catalyst performance of the obtained catalyst proper, and the like, the ratio (molar ratio) of $H_2O_2$ to Nb is preferably 0.5 to 20, and more preferably 1 to 10.

It is conventionally considered to be important that in order to properly adjust the shape and strength of a catalyst, and efficiently adjust the catalyst, the solid metal component concentration of an aqueous mixed liquid (B) just before the step (c) is not too low. As a result of studies by the present inventors, however, it has become clear that when the solid metal component concentration of an aqueous mixed liquid (B) just before the step (c) is 2.0 to 8.0% by mass, the yield of an unsaturated nitrile is rather improved.

Therefore, from the viewpoints of the shape and strength of a catalyst, and of the improvement of the yield of an unsaturated nitrile, the solid metal component concentration of an aqueous mixed liquid (B) is 2.0 to 8.0% by mass, preferably 2.5 to 7.5% by mass, more preferably 3.0 to 7.0% by mass, and still more preferably 3.5 to 6.5% by mass. If the solid metal component concentration is less than 2.0% by mass, the shape and strength of a catalyst particle decreases. By contrast, if the solid metal component concentration exceeds 8.0% by mass, since the solubility of an Sb raw material becomes poor and the metal component is not well dispersed on a carrier, the catalyst performance decreases. Here, the term "solid metal component" refers to a component obtained by excluding a carrier component from a solid component in an aqueous mixed liquid (B). The term "solid metal component concentration" refers to a value calculated by multiplying a value of a mass of a solid metal component divided by a mass of an aqueous mixed liquid (B), by 100.

Further from the viewpoint of making the shape and strength of a catalyst particle and the catalyst performance proper, and the like, the solid component concentration of an aqueous mixed liquid (B) is preferably 6.0 to 20% by mass, more preferably 6.5 to 19.5% by mass, still more preferably 7.0 to 19% by mass, and further still more preferably 7.5 to 18.5% by mass. The solid component concentration of an aqueous mixed liquid (B) is 6.0% by mass or more, and thereby the catalyst shape, the activity, and the unsaturated nitrile yield tend to be further improved. Further the solid component concentration of an aqueous mixed liquid (B) is 20% by mass or less, and thereby the activity and the unsaturated nitrile yield tend to be further improved. Here, the term "solid component concentration" refers to a value calculated by multiplying a value of a mass of a solid component divided by a mass of an aqueous mixed liquid (B), by 100.

Method for measuring the solid component concentration and the solid metal component concentration of an aqueous mixed liquid (B) are not particularly limited, but for example, the inductively coupled plasma atomic emission spectroscopy (ICP) can be used. The solid component concentration of an aqueous mixed liquid (B) is calculated by the following method. The mass of the aqueous mixed liquid (B) is measured, and thereafter, the aqueous mixed liquid (B) is evaporated to dryness at 100°C to obtain a whole solid component ($\delta$) in the aqueous mixed liquid (B). The mass of the whole solid component ($\delta$) is measured. Then, the solid component concentration of the aqueous mixed liquid (B) is calculated by dividing a mass ($M_\delta$) of the whole solid component ($\delta$) by a mass ($M_D$) of the aqueous mixed liquid (B) and multiplying the quotient by 100.

$$\texttt{Solid component concentration (\%) = } M_\delta/M_D \times 100$$

Further the solid metal component concentration of an aqueous mixed liquid (B) is calculated as follows. By measuring an aqueous mixed liquid (B) or an aqueous mixed liquid (B) diluted into a predetermined concentration by using ICP, respective concentrations X (mg/L) of elements (Si, Al, Zr and the like) of a carrier component can be calculated. Here, the volume of a solution to be used for the measurement is taken to be V (L). At this time, the mass ($M_T$) of an oxide (for example, $SiO_2$ for Si) of the each carrier element in the aqueous mixed liquid (B) is calculated by the following formula.

$$\texttt{A mass of an oxide of each carrier element in an}$$
$$\texttt{aqueous mixed liquid } (M_T) \texttt{ = X (mg/L)} \times \texttt{V (L)} \times \texttt{1000} \times \texttt{a}$$
$$\texttt{molecular weight of the oxide of the each carrier element}$$
$$\texttt{(g) / an atomic weight of the each carrier element (g)}$$

Thereafter, the solid metal component concentration can be calculated by dividing a value obtained by subtracting a mass ($M_T$) of the oxides of the carrier elements in the aqueous mixed liquid (B) from the mass ($M_\delta$) of the whole solid component in the aqueous mixed liquid (B), by the mass ($M_D$) of the aqueous mixed liquid (B), and multiplying the quotient by 100.

$$\texttt{Solid metal component concentration (\%) = } (M_\delta \texttt{ -}$$
$$M_T)/M_D \times 100$$

A method for measuring a concentration of a catalyst-constituting element is not particularly limited. A general method of measuring a metal concentration can be adopted. For example, X-ray fluorescence analysis (XRF) can be used. When a concentration of a metal in a solid particle catalyst is measured, the XRF can be suitably used from the viewpoints of simplicity of measurement and precision of quantitative determination or the like. When a slight amount of metal is analyzed, a catalyst is dissolved in an appropriate solution, and the amount can be determined by ICP or atomic absorption using the solution liquid. When the amounts of carbon, hydrogen, and nitrogen are desired to be determined, CHN analysis can be suitably used.

In the step (a) and/or (b), a raw material comprising W (hereinafter, also referred to as "W raw material") may further be mixed. The W raw material is not particularly limited. Specifically, a tungsten salt such as an ammonium salt, a nitrate, a carboxylate, ammonium carboxylate, a peroxocarboxylate, ammonium peroxocarboxylate, a halogenated ammonium salt, a halide, acetylacetonate, an alcoxide, a triphenyl compound, a polyoxometalate, ammonium polyoxometalate; tungsten trioxide, tungsten dioxide, tungstic acid, an ammonium metatungstate aqueous solution, ammonium paratungstate, tungstosilicic acid, silicotungstomolybdic acid, and tungstosilicic acid or the like can be used. Among them, the ammonium metatungstate aqueous solution is preferable.

In the step (a) and/or (b), there may further be mixed a raw material (hereinafter, also referred to as "Z raw material") comprising one or more elements (hereinafter, also referred to as "component Z") selected from the group consisting of La, Ce, Pr, Yb, Y, Sc, Sr and Ba. The Z raw material is not particularly limited as long as being a substance comprising a component Z, and there can be used a compound comprising a component Z, or metals of a Z component solubilized by an appropriate reagent. The compound comprising a component Z is not particularly limited, and there can be used, for example, an ammonium salt, a nitrate, a carboxylate, an ammonium carboxylate, a peroxocarboxylate, an ammonium peroxocarboxylate, a halogenated ammonium salt, a halide, acetylacetonate, or an alkoxide. Among them, an aqueous raw material such as a nitrate or a carboxylate is preferably used.

In the step (a) and/or (b), the raw material ratio is preferably adjusted so that the oxide catalyst obtained in the step (d) has a composition ratio indicated by the following composition formula (1). The oxide catalyst satisfies the following composition formula (1), and thereby the yield of an unsaturated nitrile is further improved.

$$Mo_1V_aSb_bNb_cW_dZ_eO_n \ldots \qquad (1),$$

wherein the component Z is at least one element selected from the group consisting of La, Ce, Pr, Yb, Y, Sc, Sr and Ba; a, b, c, d, e and n represent atomic ratios of the elements; and $0.10 \leq a < 0.20$, $0.15 \leq b \leq 0.30$, $0.010 \leq c \leq 0.50$, $0 \leq d \leq 0.40$, and $0 \leq e \leq 0.20$.

a and b in the above composition formula (1) preferably satisfies $0.5 \leq a/b \leq 0.98$, more preferably $0.55 \leq a/b \leq 0.97$, and still more preferably $0.60 \leq a/b \leq 0.96$. The solubility of an Sb compound as a raw material to water is lower than that of other catalyst-constituting metal components. On the other hand, by oxidizing Sb by V in an aqueous solution, the solubility of the Sb compound can be improved. Therefore, from the viewpoint of homogenizing the metal component in a slurry, it is usually desirable that V is in a relatively large amount to Sb. However, with a method for producing an oxide catalyst according to the present embodiment, the metal component in the slurry can be homogenized even under the condition that the ratio of V/Sb of a catalyst composition, that is, a/b, is low, and V in order to dissolve Sb is in a relatively small amount to Sb.

This composition ratio may be a value different from a composition ratio of an oxide catalyst to be finally obtained. This is because a protrusion of a catalyst to be described later has a composition different from that of the main body of the catalyst; the composition ratio of the whole catalyst is also changed by removing the protrusion from the main body of the catalyst. Therefore, in the step (a) and/or (b) step the composition ratio is set in consideration of the change. In the present specification, the term "protrusion" means an object oozing and/or adhering on the surface of a calcined body obtained by main calcination to be described later. The term "protrusion" means an object projecting from the surface of the calcined body or adhering on the surface.

Hereinafter, the above step (a) and/or (b) will be described using an example in which an aqueous mixed liquid (B) of a silica-supported catalyst comprising a Mo raw material, a V raw material, an Sb raw material, a Nb raw material, a W raw material and a Z raw material using water as a solvent and/or a dispersion medium. Note that the step (a) and/or (b) is not limited thereto.

The Mo raw material, the V raw material, the Sb raw material and the Z raw material are added to water, and heated to prepare an aqueous mixed liquid (A). A heating temperature and a heating time when the aqueous mixed liquid (A) is prepared are preferably adjusted such that the each raw material can be sufficiently dissolved. Specifically, the heating temperature is preferably 70°C to 100°C; and the heating time is preferably 30 minutes to 5 hours. At this time, the aqueous mixed liquid (A) is preferably stirred such that the raw materials are likely to be dissolved. The solid component concentration of the aqueous mixed liquid (A) is preferably 3 to 9% by mass. At this time, the preparation atmosphere of the aqueous mixed liquid (A) may be an air atmosphere, but may be a nitrogen atmosphere from the viewpoint of adjusting the oxidation number of an obtained oxide catalyst. The state of the aqueous mixed liquid (A) after heating is ended is defined as an aqueous mixed liquid (A'). The temperature of the aqueous mixed liquid (A') is preferably held at 20°C or more and 80°C or less, and more preferably 40°C or more and 80°C or less. The temperature of the aqueous mixed liquid (A') is 20°C or more, and thereby the deposition of the metal species dissolved in the aqueous mixed liquid (A') tends to be unlikely to occur.

Then, a silica sol is added to an aqueous mixed liquid (A) or an aqueous mixed liquid (A'). Between these, a silica sol is preferably added to the aqueous mixed liquid (A'). The silica sol functions as a carrier. A temperature when the silica sol is added is preferably 80°C or less. When the silica sol is added at 80°C or less, the stability of the silica sol is comparatively high, which tends to suppress the gelling of the aqueous mixed liquid (B). The silica sol may be added may when aging to be described later is started, in the middle of aging, or just before the aqueous mixed liquid (B) is dried.

Further, from the viewpoint of adjusting the oxidation number of the obtained composite oxide, an appropriate amount of hydrogen peroxide water is preferably added to the aqueous mixed liquid (A) or the aqueous mixed liquid (A') if needed. The hydrogen peroxide water may be added to the aqueous mixed liquid (A) or the aqueous mixed liquid (A') itself, in the middle of preparing the aqueous mixed liquid (A) or the aqueous mixed liquid (A'), or before or after the silica sol is added. At this time, from the viewpoint of adjusting the oxidation number of the obtained oxide catalyst to a proper range, as for the amount of a hydrogen peroxide water to be added, $H_2O_2/Sb$ (molar ratio) is preferably 0.01 to 5, more preferably 0.5 to 3, and still more preferably 1 to 2.5.

The heating temperature and the heating time after the hydrogen peroxide water is added to the aqueous mixed liquid (A') or the aqueous mixed liquid (A') are preferably such that a liquid phase oxidation reaction due to the hydrogen peroxide water can sufficiently proceed. Specifically, the heating temperature is preferably 20°C to 80°C, and the heating time is preferably 5 minutes to 4 hours. Similarly, the rotation number of stirring during heating can be adjusted to an appropriate rotation number in which the liquid phase oxidation reaction due to the hydrogen peroxide water is likely to proceed. From the viewpoint of causing the liquid phase oxidation reaction due to the hydrogen peroxide water to sufficiently proceed, a stirring state is preferably kept during heating. An aqueous mixed liquid thus prepared by adding a hydrogen peroxide water is defined as (A'').

Next, as a Nb raw material, a mixed liquid $(B_0)$ is preferably prepared by heating while stirring a Nb raw material and a dicarboxylic acid in water. The dicarboxylic acid is not particularly limited, but an example thereof includes oxalic acid $[(COOH)_2]$. Then, hydrogen peroxide water is preferably added to the mixed liquid $(B_0)$ to prepare an aqueous mixed liquid $(B_1)$. At this time, from the viewpoints of forming a complex with the Nb raw material to stabilize the Nb raw material in a dissolved state, of properly adjusting the oxidation/reduction state of the catalyst-constituting element, and of making the catalyst performance of the obtained catalyst proper, and the like, $H_2O_2/Nb$ (in molar ratio) is preferably 0.5 to 20, and more preferably 1 to 10.

Then, depending on a composition to be targeted, one of the aqueous mixed liquids (A), (A') and (A''), and one of the aqueous mixed liquids $(B_0)$ and $(B_1)$ are mixed to obtain an aqueous mixed liquid (B). At this time, a W raw material and a powder silica may further be mixed.

From the viewpoint of making the catalyst performance proper, the powder silica is preferably added to the "aqueous mixed liquid (A'')" or a "solution obtained by mixing the aqueous mixed liquid (B) with a W raw material". The powder silica can be added as it is. More preferably, the powder silica is preferably added as a liquid in which powder silica is dispersed in water, i.e., a powder silica-containing suspension liquid. The concentration of the powder silica in the powder silica-containing suspension liquid at this time is preferably 1 to 30% by mass, and more preferably 3 to 20% by mass. The concentration of the powder silica is 1% by mass or more, and thereby the distorted shape of the catalyst particle caused by the low viscosity of the aqueous mixed liquid (B) tends to be able to be suppressed. The occurrence or the like of a depression in the catalyst particle also tends to be able to be suppressed. The concentration of the powder silica is 30% by mass or less, and thereby the gelling of the aqueous mixed liquid (B) and clogging a pipeline caused by the high viscosity of the aqueous mixed liquid (B) tend to be able to be avoided, making it possible to easily obtain a dried powder. Further, the catalyst performance also tends to be further improved.

In a step (c) to be described later, the aqueous mixed liquid (B) is prepared such that the solid metal component concentration of the aqueous mixed liquid (B) is 2.0 to 8.0% by mass. In addition, the aqueous mixed liquid (B) is preferably prepared such that the solid component concentration of the aqueous mixed liquid (B) is 6.0 to 20% by mass. A method for adjusting the solid metal component concentration and the solid component concentration is not particularly limited, and examples thereof include increasing and/or decreasing the amount of water, the concentration of the silica sol, the concentration of the powder silica dispersion liquid, and the like used to prepare aqueous mixed liquid $A_1$.

Then, the obtained aqueous mixed liquid (B) may be subjected to an aging treatment. The aging of the aqueous mixed liquid (B) means that the aqueous mixed liquid (B) is left standstill or stirred for a predetermined time. An aging time is preferably 90 minutes or more and 50 hours or less, and more preferably 90 minutes or more and 6 hours or less. The aging time is within the above-mentioned range, and thereby the aqueous mixed liquid (B) having a suitable oxidation/reduction state (electric potential) is likely to be formed, which tends to further improve the catalyst performance of the obtained composite oxide.

Here, when the oxide catalyst is industrially produced after drying by a spray dryer, the treatment speed of the spray dryer is ordinarily rate-controlling. A long time is required until the spray drying of the whole mixed liquid is ended after a part of the aqueous mixed liquid (B) is spray-dried. In the meantime, the aging of the aqueous mixed liquid which is not subjected to spray drying treatment is continued. Therefore, the aging time includes not only an aging time before drying in a step (c) to be described later but also a time from the starting to ending of drying.

From the viewpoint of preventing the condensation of an Mo component and the deposition of metal oxides due to V and the other metal species or a plurality of metals, an aging temperature is preferably 25°C or more. Further from the viewpoint of preventing the hydrolysis of a complex comprising Nb and hydrogen peroxide from excessively occurring to form an aqueous mixed liquid (B) in a preferable form, the aging temperature is preferably 65°C or less.

From the above viewpoint, the aging temperature is preferably 25°C or more and 65°C or less, and more preferably 45°C or more and 60°C or less. The catalyst can be further reduced during calcining by extending the aging time, raising the aging temperature or the like, or performing the extending and the raising in combination.

The present inventors have studied diligently, and, as a result, found that the reduction rate of the catalyst after calcining and the oxidation-reduction potential of the aqueous mixed liquid (B) have a certain correlation. If the oxidation-reduction potential of the aqueous mixed liquid (B) increases, the catalyst after calcining becomes oxidative. If the oxidation-reduction potential of the aqueous mixed liquid (B) decreases, the catalyst after calcining becomes reductive. Therefore, the oxidation-reduction potential of the aqueous mixed liquid (B) is preferably 400 to 600 mV, more preferably 450 to 550 mV, and still more preferably 470 to 510 mV. The oxidation-reduction potential of the aqueous mixed liquid (B) can be measured by using a commercially available potentiometer, though not particularly limited thereto.

[Step (c)]

The step (c) is a step of drying the aqueous mixed liquid (B) to obtain a dried powder. The drying can be performed by a known method, and can also be performed, for example, by spray drying or evaporation to dryness. When a fluidized-bed reaction method is adopted in the vapor-phase catalytic oxidation reaction or the vapor-phase catalytic ammoxidation reaction, a dried powder in a minute sphere state is preferably obtained from the viewpoint of setting flowability within a reactor to a preferable state or the like. Therefore, the spray drying is preferably adopted. The atomization in the spray drying method may be carried out by a centrifugation method, a two-fluid nozzle method, or a high-pressure nozzle method. Air heated by steam or an electric heater or the like may be used as the heat source for drying.

A spray velocity, a velocity of the aqueous mixed liquid (B) to be fed, and a rotation number of an atomizer in the case of the centrifugation method, or the like are preferably adjusted such that the size of the obtained dried powder is suitable. The mean particle diameter of the dried powder is preferably 35 to 75 $\mu$m, more preferably 40 to 70 $\mu$m, and still more preferably 45 to 65 $\mu$m. The mean particle diameter does not vary greatly even after calcining.

[Step (d)]

The step (d) is a step of calcining a dried powder to obtain an oxide catalyst. For example, a rotary furnace (rotary kiln) can be used as a calcining apparatus for calcining the dried powder. The shape of a calcining machine for calcining the dried powder therein is not particularly limited. The shape is preferably a tube shape (calcining tube) from the viewpoint of the fact that continuous calcining can be carried out, and particularly preferably a cylindrical shape. From the viewpoint of being likely to adjust a calcining temperature to a preferable rising temperature pattern or the like, a heating method is preferably an outer heating method. An electric furnace can be suitably used. The size and quality or the like of the calcining tube can be appropriately selected according to a calcining condition or a production amount.

In the step (d), the calcination is desirably performed in two steps. When the first calcination is defined as pre-stage calcination and the subsequent calcination is defined as main calcination, it is preferable that the pre-stage calcination is performed in the temperature range of 250 to 400°C, and the main calcination is performed in the temperature range of 450 to 700°C. The pre-stage calcination and the main calcination may be continuously carried out. The main calcination may be carried out after the pre-stage calcination is completed once. The pre-stage calcination and the main calcination may be respectively divided into several steps.

The calcination may be performed in an atmospheric environment or in a circulation of air. However, from the viewpoint of adjusting the calcining atmosphere to a preferable oxidation/reduction state, at least a part of the calcination is preferably carried out while an inert gas which is substantially free from oxygen such as nitrogen is circulated. When the calcination is performed by a batch method, the amount of the inert gas to be supplied is preferably 50 NL/hr or more per 1 kg of the dried powder from the viewpoint of adjusting the calcining atmosphere to a preferable oxidation/reduction state, more preferably 50 to 5000 NL/hr, and still more preferably 50 to 3000 NL/hr. Here, the term "NL" means a volume of a gas measured under standard temperature and pressure conditions, i.e., at 0°C under a pressure condition of 1 atmosphere.

The reduction rate of the preliminarily calcined body is preferably 7 to 15%, more preferably 8 to 12%, and still more preferably 9 to 12%. The reduction rate is within this range, and thereby the activity of the oxide catalyst is further improved and the catalyst production efficiency tends to be further improved. Specific examples of a method for controlling the reduction rate to a desired range include a method for changing the pre-stage calcination temperature, a method for adding an oxidizing component such as oxygen into an atmosphere during calcining, or a method for adding a reducing component into an atmosphere during calcining. They may be combined.

[Step (e)]

The step (e) is a step of removing a protrusion which is present on the particle surface of the oxide catalyst. Many protrusions are projecting oxide crystals and other impurities. Particularly, in the case of the calcined body comprising a plurality of metals, the oxide having a composition different from that of the crystal forming a large part of the calcined body may be formed in such a shape that the oxide oozes out from the main part of the calcined body. Such a protrusion becomes a factor of decreasing flowability. Therefore, by removing the protrusions from the catalyst surface, the catalyst performance tends to be raised. When the protrusion is removed on a gram scale, the following apparatus can be used. That is, a perpendicular tube can be used, wherein a perforated plate having one or more holes is provided in a bottom part, and a paper filter is provided in an upper part. The calcined bodies are put in the perpendicular tube, and air is circulated from a lower part. Therefore, an air current flows from each hole, to urge the contact of the calcined bodies, thereby removing the protrusions bodies.

[Oxide Catalyst]

An oxide catalyst obtained by the above production method preferably comprises metal components satisfying the following composition formula (1):

$$Mo_1V_aSb_bNb_cW_dZ_eO_n ... \qquad (1),$$

wherein the component Z is at least one element selected from the group consisting of La, Ce, Pr, Yb, Y, Sc, Sr and Ba; a, b, c, d, e and n represent atomic ratios of the elements; and $0.10 \leq a < 0.20$, $0.15 \leq b \leq 0.30$, $0.010 \leq c \leq 0.50$, $0 \leq d \leq 0.40$, and $0 \leq e \leq 0.20$.

Further, a and b in the above composition formula (1) preferably satisfies $0.5 \leq a/b \leq 0.98$, more preferably $0.55 \leq a/b \leq 0.97$, and still more preferably $0.60 \leq a/b \leq 0.96$. a/b is in the above range, and thereby the yield of a target product tends to be further improved.

[Method for Producing Unsaturated Nitrile]

A method for producing an unsaturated nitrile according to the present embodiment comprises a step of subjecting propane or isobutane to a vapor-phase catalytic oxidation reaction or a vapor-phase catalytic ammoxidation reaction with an oxide catalyst produced by the above production method of the oxide catalyst, to produce a corresponding unsaturated nitrile. Hereinafter, a method for producing acrylonitrile by using the oxide catalyst filled in a reactor will be described.

(feed stocks)

Propane and ammonia as feed stocks are not necessarily highly pure but those of industrial grade such as propane comprising 3 vol% or less of impurities, for example, ethane, ethylene, n-butane, and isobutane, and ammonia comprising about 3 vol% or less of impurities, for example, water can be used. There may be supplied to the reaction, as an oxygen-containing gas, air, air enriched with oxygen, pure oxygen, or a gas diluted with inert gas such as helium, argon, carbon dioxide or nitrogen, or water vapor. When the gases are used on an industrial scale, among them, the air is preferably used from simplicity.

(Reaction Condition)

In the vapor-phase catalytic oxidation reaction of propane or isobutane, specifically, propane or isobutane, and oxygen are allowed to react in the presence of the above oxide catalyst. The reaction condition is not particularly limited, but includes the following condition. A molar ratio of oxygen to be supplied for the reaction to propane or isobutane is preferably 0.1 to 6, and more preferably 0.5 to 4. A reaction temperature is preferably 300 to 500°C, and more preferably 350 to 500°C. A reaction pressure is preferably $5 \times 10^4$ to $5 \times 10^5$ Pa, and more preferably $1 \times 10^5$ to $3 \times 10^5$ Pa. A contact time is preferably 0.1 to 10 (sec·g/cm$^3$), and more preferably 0.5 to 5 (sec·g/cm$^3$). By making the reaction condition in the above ranges, the generation of by-products tends to be further suppressed and the yield of an unsaturated nitrile tends to be further improved.

In the present embodiment, the contact time is defined by the following formula.

$$\text{Contact Time (sec·g/cm}^3) = (W/F) \times 273/(273 + T)$$

Here, W, F, and T are defined as follows:

W = filled amount (g) of a catalyst;
F = flow rate (N cm$^3$/sec) of a feed stock mixed gas under standard conditions (0°C, 1.013 $\times$ 10$^5$ Pa); and
T = reaction temperature (°C).

In the vapor-phase catalytic ammoxidation reaction of propane or isobutane, specifically, propane or isobutane, oxygen, and ammonia are allowed to react in the presence of the above oxide catalyst. The reaction condition is not particularly limited, but includes the following condition. A molar ratio of oxygen to be supplied for the reaction to propane or isobutane is preferably 0.1 to 6, and more preferably 0.5 to 4. A molar ratio of ammonia to be supplied for the reaction to propane or isobutane is preferably 0.3 to 1.5, and more preferably 0.7 to 1.2. A reaction temperature is preferably 320 to 500°C, and more preferably 370 to 460°C. A reaction pressure is preferably 5 $\times$ 10$^4$ to 5 $\times$ 10$^5$ Pa, and more preferably 1 $\times$ 10$^5$ to 3 $\times$ 10$^5$ Pa·. A contact time is preferably 0.1 to 10 (sec·g/cm$^3$), and more preferably 0.5 to 5 (sec·g/cm$^3$) By making the reaction condition in the above ranges, the generation of by-products tends to be able to be further suppressed, and the yield of the unsaturated nitrile tends to be able to be further improved.

Conventional methods such as a fixed bed method, a fluidized bed method, and a moving bed method can be adopted as a reaction method in the vapor-phase catalytic oxidation reaction and the vapor-phase catalytic ammoxidation reaction. Among them, due to easiness of removal of a reaction heat, a fluidized bed reactor is preferable. The vapor-phase catalytic ammoxidation reaction may either be a single current system or a recycle system.

Examples

Hereinafter, the present embodiment will be described in more detail with reference to Examples and Comparative Examples, but the present embodiment is not limited to these Examples.

[Yield of Acrylonitrile (Unsaturated Nitrile)]

In Examples and Comparative Examples, the yield of acrylonitrile is based on the following definition. The molar number of the generated acrylonitrile was measured by previously analyzing a gas of acrylonitrile having a known concentration with gas chromatography (GC: manufactured by Shimadzu Corporation, product name: GC2014) to obtain a calibration curve, and thereafter injecting a certain amount of gas generated in an ammoxidation reaction into the GC.

$$\text{Yield of Acrylonitrile (\%) = (Molar Number of Generated Acrylonitrile)/(Molar Number of Supplied Propane)} \times 100$$

[Oxidation-Reduction Potential of Aqueous Mixed Liquid (B)]

The oxidation-reduction potential of an aqueous mixed liquid (B) was measured using a commercially available potentiometer (manufactured by DKK-TOA CORPORATION), though not particularly limited.

[Activity]

The activity of a catalyst is based on the following definition. The molar number of unreacted propane was measured by injecting a certain amount of gas after the ammoxidation reaction to GC.

$$\text{Activity of a catalyst} = -\ln(\text{a molar number of}$$

$$\text{unreacted propane (\%)}/100) \times 3.6(10^3 \text{ sec/h})/\text{a contact}$$

$$\text{time (sec·g/cm}^3) \times \text{a bulk specific gravity of the catalyst}$$

$$(\text{g/cm}^3)$$

[Solid Component Concentration]

The solid component concentration was calculated by dividing a mass ($M_\delta$) of the whole solid component ($\delta$) by a mass ($M_D$) of an aqueous mixed liquid (B) and multiplying the divided value by 100. The mass ($M_\delta$) of the whole solid component ($\delta$) was determined as a mass of a residue after volatile components in the aqueous mixed liquid (B) were all vaporized.

$$\text{Solid component concentration (\%)} = M_\delta/M_D \times 100$$

[Solid Metal Component Concentration]

The solid metal component concentration was calculated by dividing a value obtained by subtracting a mass ($M_T$) of an oxide of a carrier element in an aqueous mixed liquid (B) from the mass ($M_\delta$) of the whole solid component in the aqueous mixed liquid (B), by the mass ($M_D$) of the aqueous mixed liquid (B), and multiplying the divided value by 100.

$$\text{Solid metal component concentration (\%)} = (M_\delta - M_T)/M_D \times 100$$

Here, the mass ($M_T$) of the oxide of the carrier element is calculated according to the following procedure from the charging amount. The charging amount of a raw material comprising elements (Si, Al, Zr and the like) of the carrier component is taken to be N (g); and the purity, to be P (%). At this time, the mass ($M_T$) of an oxide (for example, $SiO_2$ for Si) of a carrier element is calculated by the following formula.

$$\text{A mass of an oxide of each carrier element in an aqueous mixed liquid } (M_T) = N \text{ (g)} \times P \text{ (\%)}/100$$

If the charging amount is unclear, the mass ($M_T$) of the oxide of the carrier element is calculated according to the following procedure. By measuring an aqueous mixed liquid (B), or an aqueous mixed liquid (B) diluted into a predetermined concentration by using ICP, respective concentrations X (mg/L) of elements (Si, Al, Zr and the like) of the carrier component can be calculated. Here, the volume of a solution to be used for the measurement is taken to be V (L). At this time, the mass ($M_T$) of an oxide (for example, $SiO_2$ for Si) of the each carrier element in an aqueous mixed liquid (B) is calculated by the following formula.

$$\text{A mass of an oxide of each carrier element in an aqueous mixed liquid } (M_T) = X \text{ (mg/L)} \times V \text{ (L)} \times 1000 \times \text{a molecular weight of the oxide of the each carrier element (g)} / \text{an atomic weight of the each carrier element (g)}$$

[Example 1]

(Preparation of Niobium Mixed Liquid)

A niobium mixed liquid was prepared by the following method.

1.650 kg of niobic acid containing 79.8% by mass of $Nb_2O_5$ and 6.354 kg of oxalic acid dihydrate [$H_2C_2O_4 \cdot 2H_2O$] were mixed in 10 kg of water. The molar ratio of oxalic acid/niobium charged was 5.1, and the concentration of niobium charged was 0.55 (mol-Nb/kg-liquid). This liquid was heated and stirred at 95°C for 2 hours to obtain a mixed liquid in which niobium was dissolved. The mixed liquid was allowed to stand still, and cooled with ice, and thereafter, the solid was filtered away by suction filtration to obtain a homogeneous niobium mixed liquid. The molar ratio of oxalic acid/niobium of the niobium mixed liquid was 2.420 by the following analysis.

10 g of the niobium mixed liquid was precisely weighed in a crucible, dried at 95°C overnight, and thereafter heat treated at 600°C for 1 hour to obtain 0.8154 g of $Nb_2O_5$. From the result, the niobium concentration was 0.613 (mol-Nb/kg-liquid). 3 g of the niobium mixed liquid was precisely weighed in a 300-mL glass beaker; 200 mL of hot water of about 80°C was added; and then, 10 mL of a 1:1 sulfuric acid was added. The obtained mixed liquid was titrated using a 1/4N $KMnO_4$ under stirring with the liquid being held at a liquid temperature of 70°C on a hot stirrer. The point where a faint pale pink by $KMnO_4$ continued for about 30 sec or longer was taken as an end point. The concentration of oxalic acid was calculated from the titration amount by the following formula, and was 1.485 (mol-oxalic acid/kg).

$$2KMnO_4 + 3H_2SO_4 + 5H_2C_2O_4 \rightarrow K_2SO_4 + 2MnSO_4 + 10CO_2 + 8H_2O$$

The obtained niobium mixed liquid was used as a niobium raw material liquid ($B_0$) in production of oxide catalysts of the following Examples 1 to 5.

(Preparation of Dried Powder)

A dried powder ($C_1$) was produced as follows. To 2202 g of water, 132.3 g of ammonium heptamolybdate [$(NH_4)_6Mo_7O_{24} \cdot 4H_2O$], 16.6 g of ammonium metavanadate [$NH_4VO_3$], 23.6 g of diantimony trioxide [$Sb_2O_3$], and 2.5 g of cerium nitrate [$Ce(NO_3)_3 \cdot 6H_2O$] were added and heated at 95°C for 1 hour with stirring, to prepare an aqueous mixed liquid ($A_1$).

To 129.5 g of a niobium mixed liquid in which a molar ratio of oxalic acid/niobium was 2.420, 18.2 g of hydrogen peroxide water containing 30% by mass of $H_2O_2$ was added, and mixed at room temperature for 10 minutes with stirring to prepare an aqueous mixed liquid ($A_2$).

After the obtained aqueous mixed liquid ($A_1$) was cooled to 70°C, 229.6 g of silica sol containing 34.1% by mass of $SiO_2$ was added thereto, and 18.2 g of hydrogen peroxide water containing 30% by mass of $H_2O_2$ was further added thereto. Then, the resultant mixture was continuously stirred for 30 minutes at 55°C. Next, the aqueous mixed liquid ($A_2$), 14.2 g of an ammonium metatungstate aqueous solution (purity: 50%), and a dispersion liquid obtained by dispersing 77.5 g of powder silica in 700.4 g of water were sequentially added to the aqueous mixed liquid ($A_1$), and the resultant mixture was then stirred and aged at 50°C for 2.5 hours to obtain a slurry aqueous mixed liquid ($B_1$).

The obtained aqueous mixed liquid ($B_1$) was supplied to a centrifugal spray dryer (a drying heat source is air; and the same will apply hereafter) and dried to obtain a dried powder ($C_1$) in a minute sphere state. Temperatures at an inlet and an outlet of the dryer were respectively 210°C and 120°C.

(Classification Operation)

The obtained dried powder ($C_1$) was classified using a sieve having an opening of 25 $\mu$m to obtain a dried powder ($C_2$) as a classified material. In the obtained dried powder ($C_2$), the content rate of particles of 25 $\mu$m or less was 0.2% by mass, and the mean particle diameter was 54 $\mu$m. The content rate of particles and the mean particle diameter were measured by LS230 (trade name) manufactured by BECKMAN COULTER (the same will apply hereafter).

(Calcination of Dried Powder ($C_2$))

The obtained dried powder ($C_2$) was supplied in the supplied amount of 80 g/hr to a continuous SUS cylindrical calcining tube which has a rotary furnace diameter (inner diameter; the same will apply hereafter) of 3 inches and a length of 89 cm. A nitrogen gas of 1.5 NL/min was flowed in a direction opposed to the supply direction of the dried powder (that is, countercurrent flow; the same will apply hereafter) and the same direction (that is, concurrent flow; the same will apply hereafter) respectively in the calcining tube, and the total flow rate was set to 3.0 NL/min. The temperature of the furnace was set such that the temperature of the calcining tube was raised over 4 hours to 360°C as the maximum calcining temperature while the calcining tube was rotated at a speed of 4 rotation/min, and the temperature could be held at 360°C for 1 hour, to perform pre-stage calcination. A small amount of the pre-stage calcined body collected at the outlet of the calcining tube was sampled, and heated to 400°C in a nitrogen atmosphere. The reduction rate was then measured. The reduction rate was 10.2%. The collected pre-stage calcined body was supplied in the supplied amount of 60 g/hr to a continuous SUS cylindrical calcining tube which has a rotary furnace diameter of 3 inches and a length of 89 cm. A nitrogen gas of 1.1 NL/min was flowed in a direction opposed to the supply direction of the dried powder and the same direction respectively in the calcining tube, and the total flow rate was set to 2.2 NL/min. The temperature of the furnace was set such that the temperature could be raised to 680°C over 2 hours, held at 680°C for 2 hours, and then lowered to 600°C over 8 hours, to obtain a calcined body ($D_1$) by performing main calcination.

(Removal of protrusion)

50 g of a calcined body ($D_1$) was put in a perpendicular tube (inner diameter: 41.6 mm, length: 70 cm) wherein a perforated disk having three holes each having a diameter of 1/64 inches was provided in a bottom part and a paper filter was provided in an upper part. Then, air was circulated at room temperature towards the upper part from the lower part of the perpendicular tube via each hole, to urge the contact of the calcined bodies. An air current length in the direction in which the air current at this time flowed was 56 mm, and the mean linear speed of the air current was 332 m/s. The protrusion was not present in the oxide catalyst ($E_1$) obtained after 24 hours.

The composition of the oxide catalyst ($E_1$) obtained as described above was measured by X-ray fluorescence analysis (apparatus: manufactured by Rigaku Corporation, RINT1000 (trade name), Cr tube, tube voltage: 50 kV, tube current: 50 mA, and the same will apply hereinafter). The obtained results are shown in Table 1.

(Ammoxidation Reaction of Propane)

Propane was provided for a vapor-phase catalytic ammoxidation reaction by the following method using the oxide catalyst ($E_1$) obtained above. A Vycor glass fluidized-bed reaction tube having an inner diameter of 25 mm was filled with 35 g of the composite oxide catalyst. A mixed gas having a molar ratio of propane:ammonia:oxygen:helium of 1:1:3:18 was supplied into the reaction tube at a contact time of 3.0 (sec·g/cm$^3$) at a reaction temperature of 440°C under an atmospheric pressure as a reaction pressure. The reaction yields of acrylonitrile (AN) when a successive reaction was performed for 10 days for the catalyst are shown in Table 1.

[Example 2]

A catalyst was prepared under the same condition as in Example 1, except for altering the amount of water to be firstly added to 4579 g, to obtain an oxide catalyst ($E_1$) of Example 2. Then, the ammoxidation reaction of propane was performed by the same method as that in Example 1 using the catalysts.

[Example 3]

A catalyst was prepared under the same condition as in Example 1, except for altering the amount of the silica sol to be added to the aqueous mixed liquid ($A_1$) to 401.7 g, and adding 135.7 g of the powder silica itself in which the powder silica to be added to the aqueous mixed liquid ($A_2$) had not been dispersed in water, to obtain an oxide catalyst ($E_1$) of Example 3. Then, the ammoxidation reaction of propane was performed by the same method as that in Example 1 using the catalysts.

[Example 4]

A catalyst was prepared under the same condition as in Example 1, except for altering the amount of water to be firstly added to 5767 g, to obtain an oxide catalyst ($E_1$) of Example 4. Then, the ammoxidation reaction of propane was performed by the same method as that in Example 1 using the catalysts.

[Example 5]

(Preparation of Dried Powder)

A dried powder ($C_1$) was produced as follows. To 1251 g of water, 108.7 g of ammonium heptamolybdate [$(NH_4)_6Mo_7O_{24}\cdot4H_2O$], 13.6 g of ammonium metavanadate [$NH_4VO_3$], 21.2 g of diantimony trioxide [$Sb_2O_3$], and further 2.1 g of cerium nitrate [$Ce(NO_3)_3\cdot6H_2O$] were added and heated at 95°C for 1 hour with stirring, to prepare an aqueous

mixed liquid (A$_1$).

To 107.0 g of a niobium mixed liquid (B$_0$) in which a molar ratio of oxalic acid/niobium was 2.420, 18.2 g of hydrogen peroxide water containing 30% by mass of H$_2$O$_2$ was added, and mixed at room temperature for 10 minutes with stirring to prepare an aqueous mixed liquid (A$_2$).

After the obtained aqueous mixed liquid (A$_1$) was cooled to 70°C, 401.7 g of silica sol containing 34.1% by mass of SiO$_2$ was added thereto, and 18.2 g of hydrogen peroxide water containing 30% by mass of H$_2$O$_2$ was further added thereto, and continuously stirred at 55°C for 30 minutes. Next, the aqueous mixed liquid (A$_2$), 11.7 g of an ammonium metatungstate aqueous solution (purity: 50%), and 135.7 g of the powder silica were sequentially added to the aqueous mixed liquid (A$_1$), and the resultant mixture was then stirred and aged at 50°C for 2.5 hours to obtain a slurry aqueous mixed liquid (B$_1$).

The operations thereafter were performed under the same conditions as in Example 1 to obtain an oxide catalyst (E$_1$) of Example 5. Then, the ammoxidation reaction of propane was performed by the same method as that in Example 1 using the catalysts.

[Example 6]

A catalyst was prepared under the same condition as in Example 2, except for altering the amount of the silica sol to be added to the aqueous mixed liquid (A$_1$) to 220.4 g, and adding a dispersion liquid in which 74.4 g of the powder silica to be added to the aqueous mixed liquid (A$_2$) was dispersed in 700.4 g of water, to obtain an oxide catalyst (E$_1$) of Example 6. Then, the ammoxidation reaction of propane was performed by the same method as that in Example 1 using the catalysts.

[Comparative Example 1]

A catalyst was prepared under the same condition as in Example 1, except for altering the amount of water to be firstly added to 5767 g, altering the amount of the silica sol to be added to the aqueous mixed liquid (A$_1$) to 482.1 g, and preparing the powder silica dispersion liquid to be added to the aqueous mixed liquid (A$_2$) from 162.8 g of the powder silica and 1470.8 g of water, to obtain an oxide catalyst (E$_1$) of Comparative Example 1. Then, the ammoxidation reaction of propane was performed by the same method as that in Example 1 using the catalysts.

[Comparative Example 2]

A catalyst was prepared under the same condition as in Example 1, except for altering the amount of water to be firstly added to 63.4 g, to obtain an oxide catalyst (E$_1$) of Comparative Example 2. Then, the ammoxidation reaction of propane was performed by the same method as that in Example 1 using the catalysts.

[Comparative Example 3]

A catalyst was prepared under the same condition as in Example 1, except for altering the amount of water to be firstly added to 9157.9 g, altering the amount of the silica sol to be added to the aqueous mixed liquid (A$_1$) to 688.7 g, and preparing the powder silica dispersion liquid to be added to the aqueous mixed liquid (A$_2$) from 232.6 g of the powder silica and 700.4 g of water, to obtain an oxide catalyst (E$_1$) of Comparative Example 3. Then, the ammoxidation reaction of propane was performed by the same method as that in Example 1 using the catalysts.

[Comparative Example 4]

A catalyst was prepared under the same condition as in Example 1, except for altering the amount of water to be firstly added to 700.0 g, altering the amount of the silica sol to be added to the aqueous mixed liquid (A$_1$) to 344.3 g, and preparing the powder silica dispersion liquid to be added to the aqueous mixed liquid (A$_2$) from 116.3 g of the powder silica and 700.4 g of water, to obtain an oxide catalyst (E$_1$) of Comparative Example 4. Then, the ammoxidation reaction of propane was performed by the same method as that in Example 1 using the catalysts.

[Example 7]

A catalyst was prepared under the same condition as in Example 1, except for altering the amount of water to be firstly added to 8150 g, to obtain an oxide catalyst (E$_1$) of Example 7. Then, the ammoxidation reaction of propane was performed by the same method as that in Example 1 using the catalysts.

[Comparative Example 5]

A catalyst was prepared under the same condition as in Example 1, except for altering the amount of water to be firstly added to 1100 g, to obtain an oxide catalyst ($E_1$) of Comparative Example 5. Then, the ammoxidation reaction of propane was performed by the same method as that in Example 1 using the catalysts.

[Example 8]

(Preparation of Dried Powder)

A dried powder ($C_1$) was produced as follows. To 3125 g of water, 216.05 g of ammonium heptamolybdate [$(NH_4)_6Mo_7O_{24} \cdot 4H_2O$], 29.95 g of ammonium metavanadate [$NH_4VO_3$], 42.15 g of diantimony trioxide [$Sb_2O_3$], and further 2.4 g of cerium nitrate [$Ce(NO_3)_3 \cdot 6H_2O$] were added and heated at 95°C for 1 hour with stirring, to prepare an aqueous mixed liquid ($A_1$).

To 189.2 g of the niobium mixed liquid ($B_0$) in which a molar ratio of oxalic acid/niobium was 2.420, 33.2 g of hydrogen peroxide water containing 30% by mass of $H_2O_2$ was added, mixed at room temperature for 10 minutes with stirring to prepare an aqueous mixed liquid ($A_2$).

After the obtained aqueous mixed liquid ($A_1$) was cooled to 70°C, 403.9 g of silica sol containing 34.1% by mass of $SiO_2$ was added thereto, and 49.2 g of hydrogen peroxide water containing 30% by mass of $H_2O_2$ was further added thereto, and continuously stirred at 55°C for 30 minutes. Next, the aqueous mixed liquid ($A_2$), 7.1 g of an ammonium metatungstate aqueous solution (purity: 50%), and a dispersion liquid obtained by dispersing 105.75 g of powder silica in 1427.5 g of water were sequentially added to the aqueous mixed liquid ($A_1$), and the resultant mixture was then stirred and aged at 50°C for 2.5 hours to obtain a slurry aqueous mixed liquid ($B_1$).

The operations thereafter were performed under the same conditions as in Example 1 to obtain an oxide catalyst ($E_1$) of Example 8. Then, the ammoxidation reaction of propane was performed by the same method as that in Example 1 using the catalysts.

[Comparative Example 6]

A catalyst was prepared under the same condition as in Example 8, except for altering the amount of water to be firstly added to 778.5 g, to obtain an oxide catalyst ($E_1$) of Comparative Example 6. Then, the ammoxidation reaction of propane was performed by the same method as that in Example 1 using the catalysts.

[Example 9]

(Preparation of Dried Powder)

A dried powder ($C_1$) was produced as follows. To 2800 g of water, 409.5 g of ammonium heptamolybdate [$(NH_4)_6Mo_7O_{24} \cdot 4H_2O$], 40.4 g of ammonium metavanadate [$NH_4VO_3$], 100.9 g of diantimony trioxide [$Sb_2O_3$], and further 6.1 g of cerium nitrate [$Ce(NO_3)_3 \cdot 6H_2O$] were added and heated at 95°C for 1 hour with stirring, to prepare an aqueous mixed liquid ($A_1$).

To 385.5 g of a niobium mixed liquid ($B_0$) in which a molar ratio of oxalic acid/niobium was 2.420, 56.4 g of hydrogen peroxide water containing 30% by mass of $H_2O_2$ was added mixed at room temperature for 10 minutes with stirring to prepare an aqueous mixed liquid ($A_2$).

After the obtained aqueous mixed liquid ($A_1$) was cooled to 70°C, 711.1 g of silica sol containing 34.1% by mass of $SiO_2$ was added thereto, and 117.5 g of hydrogen peroxide water containing 30% by mass of $H_2O_2$ was further added thereto, and continuously stirred at 55°C for 30 minutes. Next, the aqueous mixed liquid ($A_2$), 47 g of an ammonium metatungstate aqueous solution (purity: 50%), and a dispersion liquid obtained by dispersing 242.5 g of powder silica in 3480.8 g of water were sequentially added to the aqueous mixed liquid ($A_1$), and the resultant mixture was then stirred and aged at 50°C for 2.5 hours to obtain a slurry aqueous mixed liquid ($B_1$).

The operations thereafter were performed under the same conditions as in Example 1 to obtain an oxide catalyst ($E_1$) of Example 9. Then, the ammoxidation reaction of propane was performed by the same method as that in Example 1 using the catalysts.

[Comparative Example 7]

A catalyst was prepared under the same condition as in Example 9, except for altering the amount of water to be firstly added to 700 g, to obtain an oxide catalyst ($E_1$) of Comparative Example 7. Then, the ammoxidation reaction of propane was performed by the same method as that in Example 1 using the catalysts.

There are shown in Table 1 the activities and the yields of the reactions using the catalysts indicated in Examples 1 to 9 and Comparative Examples 1 to 7.

[Table 1]

| | Solid Metal Component Concentration | Solid Component Concentration | Activity | Acrylonitrile Yield (%) | Catalyst Composition |
|---|---|---|---|---|---|
| Example 1 | 5.6% | 10.0% | 2.64 | 54.3 | $Mo_1V_{0.187}Sb_{0.200}Nb_{0.118}W_{0.045}Ce_{0.009}$ |
| Example 2 | 3.4% | 6.0% | 2.60 | 54.0 | |
| Example 3 | 7.9% | 18.6% | 2.57 | 53.8 | |
| Example 4 | 2.8% | 5.0% | 2.54 | 53.4 | |
| Example 5 | 7.9% | 21.0% | 2.51 | 53.2 | $Mo_1V_{0.187}Sb_{0.219}Nb_{0.118}W_{0.045}Ce_{0.009}$ |
| Example 6 | 3.4% | 5.9% | 2.58 | 53.6 | $Mo_1V_{0.187}Sb_{0.200}Nb_{0.118}W_{0.045}Ce_{0.009}$ |
| Comparative Example 1 | 1.9% | 5.0% | 2.41 | 52.1 | |
| Comparative Example 2 | 14.1% | 25.0% | 2.43 | 52.3 | |
| Comparative Example 3 | 1.8% | 6.0% | 2.46 | 52.6 | |
| Comparative Example 4 | 9.2% | 20.0% | 2.47 | 52.7 | |
| Example 7 | 2.1% | 3.7% | 2.53 | 53.2 | |
| Comparative Example 5 | 8.1% | 14.0% | 2.46 | 52.9 | |
| Example 8 | 5.7% | 10.0% | 2.70 | 56.3 | $Mo_1V_{0.207}Sb_{0.218}Nb_{0.102}W_{0.030}Ce_{0.005}$ |
| Comparative Example 6 | 9.7% | 17.1% | 2.44 | 55.5 | |
| Example 9 | 7.5% | 13.3% | 2.33 | 52.5 | $Mo_1V_{0.147}Sb_{0.255}Nb_{0.110}W_{0.030}Ce_{0.007}$ |
| Comparative Example 7 | 10.0% | 17.7% | 2.02 | 48.7 | |

According to the following condition in the case where the yield was raised by 0.5% and $CO_X$ as by-products was instead decreased by 0.5%, nearly 80,000 tons of $CO_X$ is enabled to be decreased in one year. From the viewpoint of being able to cut down $CO_X$ as greenhouse gases in such a large scale, Examples 1 to 5 attain a more advantageous effect than Comparative Examples 1 to 4.

(Condition)

World production capacity of acrylonitrile: about 6,000,000 tons/year
Before the improvement, AN yield: 53%, $CO_X$: 19%
After the improvement, AN yield: 53.5%, $CO_X$: 18.5%
The present application is based on Japanese Patent Application (Japanese Patent Application No. 2014-074011) filed with the Japan Patent Office on March 31, 2014, and the contents thereof are incorporated herein by reference.

Industrial Applicability

The method for producing an oxide catalyst according to the present invention has an industrial applicability as a method for producing an oxide catalyst to be used in production of an unsaturated nitrile.

**Claims**

1. A method for producing an oxide catalyst to be used for production of an unsaturated nitrile, the method comprising:

   a step (a) of preparing an aqueous mixed liquid (A) comprising Mo, V and Sb;
   a step (b) of mixing the aqueous mixed liquid (A), a carrier component and a Nb raw material at 20°C or more and 80°C or less to obtain an aqueous mixed liquid (B);
   a step (c) of drying the aqueous mixed liquid (B) to obtain a dried powder; and
   a step (d) of calcining the dried powder to obtain an oxide catalyst,

   wherein the aqueous mixed liquid (B) has a solid metal component concentration of 2.0 to 8.0% by mass.

2. The method for producing the oxide catalyst according to claim 1, wherein the aqueous mixed liquid (B) has a solid component concentration of 6.0 to 20% by mass.

3. The method for producing the oxide catalyst according to claim 1 or 2, wherein the carrier component comprises a silica; and an amount of the silica mixed is 20 to 70% by mass in terms of $SiO_2$ based on a total amount of the oxide catalyst.

4. The method for producing the oxide catalyst according to any one of claims 1 to 3, wherein the oxide catalyst comprises a metal component represented by the following composition formula (1):

$$Mo_1V_aSb_bNb_cW_dZ_eO_n \ldots \qquad (1),$$

   wherein the component Z is at least one element selected from the group consisting of La, Ce, Pr, Yb, Y, Sc, Sr and Ba; a, b, c, d, e and n represent atomic ratios of each element and satisfy $0.10 \leq a < 0.20$, $0.15 \leq b \leq 0.30$, $0.010 \leq c \leq 0.50$, $0 \leq d \leq 0.40$, and $0 \leq e \leq 0.20$.

5. The method for producing the oxide catalyst according to claim 4, wherein a and b in the composition formula (1) satisfy $0.5 \leq a/b \leq 0.98$.

6. A method for producing an unsaturated nitrile, comprising a step of subjecting propane or isobutane to a vapor-phase catalytic oxidation reaction or a vapor-phase catalytic ammoxidation reaction with an oxide catalyst produced by the method for producing the oxide catalyst according to any one of claims 1 to 5 to produce a corresponding unsaturated nitrile.

## INTERNATIONAL SEARCH REPORT

| | International application No. |
| --- | --- |
| | PCT/JP2015/056744 |

| A. CLASSIFICATION OF SUBJECT MATTER |
| --- |
| *B01J23/30*(2006.01)i, *B01J37/04*(2006.01)i, *C07C253/26*(2006.01)i, *C07C255/08*(2006.01)i, *C07B61/00*(2006.01)n |

According to International Patent Classification (IPC) or to both national classification and IPC

| B. FIELDS SEARCHED |
| --- |
| Minimum documentation searched (classification system followed by classification symbols)<br>B01J23/30, B01J37/04, C07C253/26, C07C255/08, C07B61/00 |

| Documentation searched other than minimum documentation to the extent that such documents are included in the fields searched |
| --- |
| Jitsuyo Shinan Koho 1922–1996 Jitsuyo Shinan Toroku Koho 1996–2015<br>Kokai Jitsuyo Shinan Koho 1971–2015 Toroku Jitsuyo Shinan Koho 1994–2015 |

| Electronic data base consulted during the international search (name of data base and, where practicable, search terms used) |
| --- |

### C. DOCUMENTS CONSIDERED TO BE RELEVANT

| Category* | Citation of document, with indication, where appropriate, of the relevant passages | Relevant to claim No. |
| --- | --- | --- |
| X<br>A | JP 2006-55681 A (Asahi Kasei Chemicals Corp.),<br>02 March 2006 (02.03.2006),<br>claims 1, 7, 8; paragraphs [0033], [0034]<br>& US 2008/0108843 A1 & WO 2006/019078 A1<br>& EP 1806178 A1 | 1-3,6<br>4,5 |
| X<br>A | JP 2013-198902 A (Saudi Basic Industries<br>Corp.),<br>03 October 2013 (03.10.2013),<br>claims 1, 5<br>& US 6037304 A & EP 1020433 A2<br>& DE 60009582 D | 6<br>1-5 |

| ☒ Further documents are listed in the continuation of Box C. | ☐ See patent family annex. |
| --- | --- |

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 30 April 2015 (30.04.15) | 19 May 2015 (19.05.15) |

| Name and mailing address of the ISA/<br>Japan Patent Office<br>3-4-3,Kasumigaseki,Chiyoda-ku,<br>Tokyo 100-8915,Japan | Authorized officer |
| --- | --- |
| | Telephone No. |

Form PCT/ISA/210 (second sheet) (July 2009)

**INTERNATIONAL SEARCH REPORT**

International application No.

PCT/JP2015/056744

C (Continuation).   DOCUMENTS CONSIDERED TO BE RELEVANT

| Category* | Citation of document, with indication, where appropriate, of the relevant passages | Relevant to claim No. |
|---|---|---|
| A | JP 2011-529777 A   (Ineos USA L.L.C.), 15 December 2011 (15.12.2011), claims 1, 10; paragraph [0069] & US 2011/0218352 A1     & WO 2010/014206 A1 & EP 2310123 A | 1-6 |
| A | US 2009/0198081 A1   (PAPARIZOS CHRISTOS), 06 August 2009 (06.08.2009), claims 1 to 24; paragraphs [0053] to [0055], [0091] & WO 2009/099538 A1 | 1-6 |
| A | JP 2013-169482 A   (Mitsubishi Rayon Co., Ltd.), 02 September 2013 (02.09.2013), claims 1, 4; paragraphs [0040] to [0055] (Family: none) | 1-6 |

Form PCT/ISA/210 (continuation of second sheet) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2000070714 A
- JP 2002219362 A
- WO 2012090979 A
- JP 2014074011 A